# EUROPEAN PATENT APPLICATION

(11) **EP 2 639 856 A1**
(43) Date of publication of application: **18.09.2013**
(21) Application number: 11840656.0
(22) Date of filing: 09.11.2011
(51) Int. Cl.: H01M 2/20, A61B 1/00, H01M 2/10

(54) **BATTERY MODULE, CAPSULE ENDOSCOPE EQUIPPED WITH BATTERY MODULE**

(30) Priority: 12.11.2010 JP 2010253957
(71) Applicant: Olympus Corporation, Shibuya-ku Tokyo 151-0072 (JP)
(72) Inventor: MAKINO, Yukiharu, Tokyo 151-0072 (JP)
(74) Representative: Winter, Brandl, Fürniss, Hübner, Röss, Kaiser, Polte - Partnerschaft
(86) International application number: PCT/JP2011/075798
(87) International publication number: WO 2012/063854

(57) **Abstract**

There is provided a battery module 100 including batteries 1 and 2 and a connection body 3 between the batteries 1 and 2 which electrically connects in series a metal electrode 1b of negative electrode of the battery 1 and a metal electrode 2a of positive electrode of the battery 2, wherein the connection body 3 includes a first connection portion 3b which is electrically connected to the metal electrode 1b of the battery 1 and a second connection portion 3 a which is electrically connected to the metal electrode 2a of the battery 2 and is arranged in a region which overlaps with the individual batteries 1 and 2 when the individual batteries 1 and 2 connected by the connection body 3 are viewed in a plan view from one of two sides in a direction S of series connection of the individual batteries 1 and 2.

## Description

### Technical Field

The present invention relates to a battery module including a plurality of batteries and a connection body which connects in series the plurality of batteries and a capsule endoscope including the battery module.

### Background Art

A battery module having a plurality of cells connected directly is well-known and is disclosed in, for example, Japanese Patent Application Laid-Open Publication No. 10-106533.

Japanese Patent Application Laid-Open Publication No. 10-106533 discloses a configuration which uses a connection body to connect two cells for forming a battery module which obtains a required outputted voltage by directly connecting the individual cells.

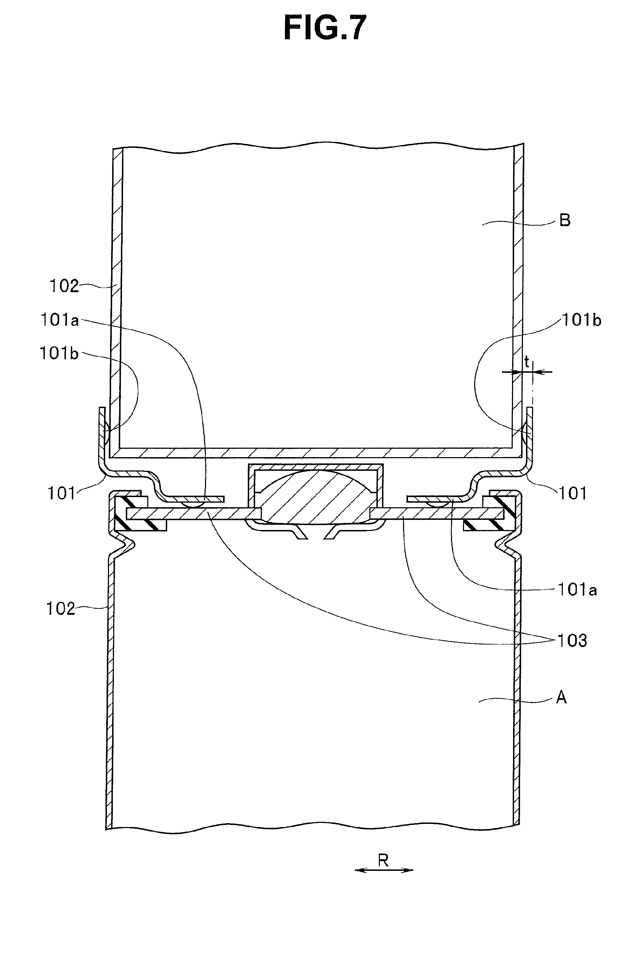

Fig. 7 shows a partial cross-sectional view showing a configuration of a conventional battery module. More specifically, as shown in Fig. 7, Japanese Patent Application Laid-Open Publication No. 10-106533 discloses a configuration of a battery module in which respective outer surfaces of batteries A and B of a same type and a same standard and in the form of a circular column are covered with a metal sheath 102 doubling as a negative electrode, except for respective one end faces (the one end face of the battery B is not shown). In the configuration, a metal electrode 103 which is insulated from the metal sheath 102 is provided as a positive electrode at each one end face of the batteries A and B (the metal electrode 103 of the battery B is not shown), and the metal electrode 103 of the battery A and the metal sheath 102 of the battery B are electrically connected by a connection body 101 to bring the batteries A and B connected in series.

Note that a connection portion 101b of the connection body 101 for the battery B is shaped to fit with the battery B and that the connection portion 101b is welded to an outer circumferential side of the metal sheath 102 of the battery B after the battery B is fitted with the connection portion 101b.

A connection portion 101a of the connection body 101 for the battery A is welded to the metal electrode 103 of the battery A. With the configuration, the connection body 101 electrically connects the batteries A and B.

However, in the configuration of the battery module disclosed in Japanese Patent Application Laid-Open Publication No. 10-106533, the connection portion 101b of the connection body 101 is welded to the outer circumferential side of the metal sheath 102 of the battery B and protrudes outward from the battery B in a radial direction R of the battery B by a thickness t corresponding to a sum of thickness of the connection portion 101b and thickness of welding flux (specifically, the thickness t of several hundred µm). As a result, reduction in diameter of a battery module is difficult, which leads to the problem of an inability to reduce size of a battery module.

Accordingly, if the battery module disclosed in Japanese Patent Application Laid-Open Publication No. 10-106533 is provided in small electronic equipment such as a capsule endoscope, the battery module interferes, by an amount corresponding to the thickness t, with reduction in diameter of the capsule endoscope.

Since inner diameter in a short axis direction of the capsule endoscope is as very small as 10 to 11 mm, if the battery module is provided in the capsule endoscope such that a radial direction of the battery module coincides with the short axis direction of the capsule endoscope, the connection body protruding only by several hundred µm interferes with reduction in diameter of the capsule endoscope.

The protrusion of the connection portion 101b by the thickness t also lowers ease of assembly of the battery module to the capsule endoscope. In addition, since the connection portion 101b is located exposed outside an outer circumference of the battery B, insulation of the connection portion 101b from other parts in the capsule endoscope needs to be separately taken into account.

There is thus a need for a configuration of a small battery module without protrusion of a connection body from a battery.

The present invention has been made in consideration of the above-described circumstances, and has as an object to provide a battery module including a configuration which allows achievement of reduction in diameter to achieve size reduction and a capsule endoscope including the battery module.

### Disclosure of Invention

### Means for Solving the Problem

In order to attain the above-described object, a battery module according to one aspect of the present invention is a battery module including a plurality of batteries formed to have a same diameter and a connection body between the plurality of batteries which electrically connects in series a metal electrode of one polarity of one of the batteries with a metal electrode of the other polarity of another battery, wherein the connection body includes a first connection portion which is electrically connected to the metal electrode of the one battery and a second connection portion which is electrically connected to the metal electrode of the other battery and is arranged in a region which overlaps with the individual batteries when the individual batteries connected by the connection body are viewed in a plan view from one of two sides in a direction of series connection of the individual batteries.

A capsule endoscope including a battery module according to one aspect of the present invention includes the battery module according to any one of claims 1 to 6.

### Brief Description of the Drawings

Fig. 1 is a view schematically showing a configuration of a battery module illustrating a first embodiment;
Fig. 2 is a top view of the battery module in Fig. 1 as viewed from direction II in Fig. 1;
Fig. 3A is a view schematically showing a state in which a plate-like member is set in a die in a process showing a method for manufacturing the battery module in Fig. 1;
Fig. 3B is a view schematically showing a state in which a second connection portion of the plate-like member in Fig. 3A deformed by the die is electrically connected to a metal electrode of the other button battery in the process showing the method for manufacturing the battery module in Fig. 1;
Fig. 3C is a view schematically showing a state in which a first connection portion of the plate-like member in Fig. 3B deformed by the die is electrically connected to a metal electrode of one button battery in the process showing the method for manufacturing the battery module in Fig. 1;
Fig. 3D is a view schematically showing a state in which the other button battery in Fig. 3C is moved until the other button battery faces the one button battery in the process showing the method for manufacturing the battery module in Fig. 1;
Fig. 4 is a view schematically showing a configuration of a battery module illustrating a second embodiment;
Fig. 5A is a view schematically showing a state in which a plate-like member is set in a die in a process showing a method for manufacturing the battery module in Fig. 4;
Fig. 5B is a view schematically showing a state in which a first connection portion of the plate-like member in Fig. 5A deformed by the die is electrically connected to a metal electrode of one button battery and a second connection portion is electrically connected to a metal electrode of the other button battery in the process showing the method for manufacturing the battery module in Fig. 4;
Fig. 5C is a view schematically showing a state in which the other button battery in Fig. 5B is moved until the other button battery faces the one button battery in the process showing the method for manufacturing the battery module in Fig. 4;
Fig. 6 is a cross-sectional view showing an outline of a configuration of a capsule endoscope where the battery module in Fig. 1 or 4 is provided; and
Fig. 7 is a partial cross-sectional view showing a configuration of a conventional battery module.

### Best Mode for Carrying Out the Invention

Embodiments of the present invention will be described below with reference to the drawings. It should be noted that the drawings are schematic and that a relationship between thickness and width of each member, a ratio among respective thicknesses of members, and the like are different from actual ones. The drawings, of course, include portions having different dimensional relationships and ratios.

### (First Embodiment)

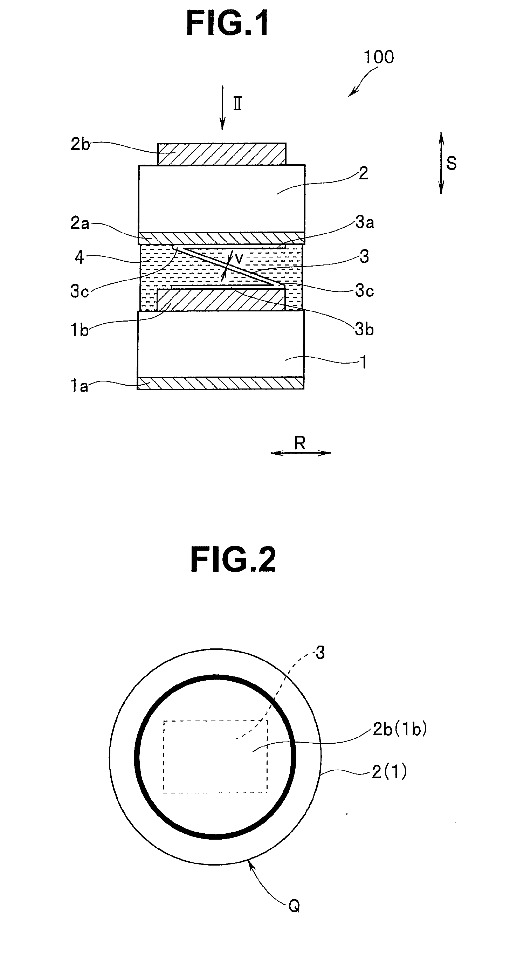

Fig. 1 is a view schematically showing a configuration of a battery module illustrating the present embodiment, and Fig. 2 is a top view of the battery module in Fig. 1 as viewed from direction II in Fig. 1.

As shown in Fig. 1, a main portion of a battery module 100 is composed of a button battery 1 as one battery, a button battery 2 as the other battery, a connection body 3, and a reinforcing agent 4.

The button battery 1 has a metal electrode 1a of positive electrode as the other polarity formed at one surface and a metal electrode 1b of negative electrode as one polarity formed at the other surface, in a direction S of series connection to the button battery 2.

The button battery 2 is of a same standard and a same type as the button battery 1, i.e., is formed to have a same diameter in a radial direction R. The button battery 2 has a metal electrode 2a of positive electrode as the other polarity formed at one surface in the direction S of series connection and a metal electrode 2b of negative electrode as one polarity formed at the other surface. That is, the metal electrode 2a is located so as to face the metal electrode 1b.

The connection body 3 is composed of a plate-like member which has a thickness v and is made of, e.g., aluminum and electrically connects in series the metal electrode 1b of the button battery 1 with the metal electrode 2a of the button battery 2.

That is, the button battery 1 and button battery 2 are formed integrally with each other by the connection body 3. In other words, the button batteries 1 and 2 are integrated together by the connection body 3. Note that the button batteries 1 and 2 do not separate after the integration by the connection body 3.

The connection body 3 causes positions in the radial direction R of the button batteries 1 and 2 formed to have the same diameter to coincide with each other to thereby integrate the button batteries 1 and 2 together.

More specifically, the button batteries 1 and 2 are integrated together such that the button battery 1 overlaps completely with the button battery 2 when the battery module 100 is viewed in a plan view from either the metal electrode 1 a of the button battery 1 or the metal electrode 2b of the button battery 2 in the direction S of series connection.

The connection body 3 includes a first connection portion 3b which is electrically connected to the metal electrode 1b and a second connection portion 3a which is electrically connected to the metal electrode 2a. The connection body 3 also has two folded portions 3c folded between the first connection portion 3b and the second connection portion 3 a, i.e., is formed by folding the plate-like member twice and has a Z-folded shape in cross section between the button batteries 1 and 2.

As shown in Figs. 1 and 2, the connection body 3 is arranged between the metal electrode 1b of the button battery 1 and the metal electrode 2a of the button battery 2 and is arranged in a region Q which overlaps with the individual batteries 1 and 2 when the battery module 100 is viewed in a plan view from, e.g., the metal electrode 2b of the button battery 2 in the direction S of series connection. That is, the connection body 3 is not arranged such that a portion of the connection body 3 protrudes in the radial direction R from outer circumferences of the button batteries 1 and 2.

The reinforcing agent 4 is composed of, e.g., epoxy resin, and the region Q is filled with the reinforcing agent 4. The reinforcing agent 4 has a function of reinforcing connection of the first connection portion 3b to the metal electrode 1b and connection of the second connection portion 3a to the metal electrode 2a.

Note that only the region Q is filled with the reinforcing agent 4 and that the reinforcing agent 4 does not protrude in the radial direction R from the outer circumferences of the button batteries 1 and 2. The reinforcing agent is not limited to epoxy resin and may be composed of acrylic resin, phenol resin, polyurethane resin, or the like.

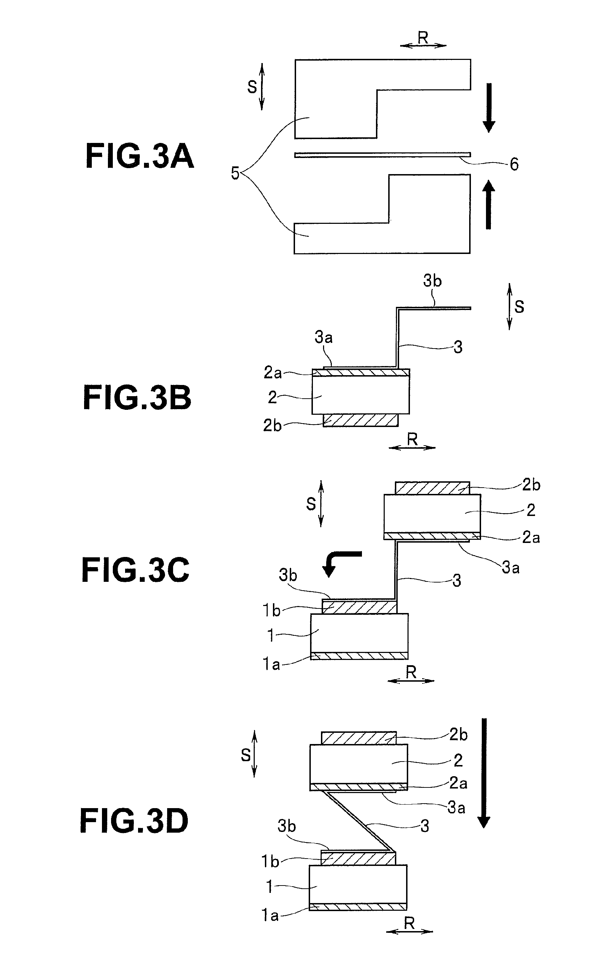

A method for manufacturing the above-described battery module 100 will be described with reference to Figs. 3A to 3D. Fig. 3A is a view schematically showing a state in which a plate-like member is set in a die in a process showing the method for manufacturing the battery module in Fig. 1. Fig. 3B is a view schematically showing a state in which a second connection portion of the plate-like member in Fig. 3A deformed by the die is electrically connected to a metal electrode of the other button battery in the process showing the method for manufacturing the battery module in Fig. 1.

Fig. 3C is a view schematically showing a state in which a first connection portion of the plate-like member in Fig. 3B deformed by the die is electrically connected to a metal electrode of one button battery in the process showing the method for manufacturing the battery module in Fig. 1. Fig. 3D is a view schematically showing a state in which the other button battery in Fig. 3C is moved until the other button battery faces the one button battery in the process showing the method for manufacturing the battery module in Fig. 1.

To manufacture the battery module 100, a plate-like member 6 made of, e.g., aluminum is first set in a die 5, as shown in Fig. 3A. After the setting, the plate-like member 6 is cranked using the die 5 so as to have two 90° folded portions. As a result, the plate-like member 6 forms the connection body 3.

As shown in Fig. 3B, the second connection portion 3a of the connection body 3 is joined to the metal electrode 2a of the button battery 2 by, e.g., a known ultrasound joining method. As a result, the second connection portion 3a is electrically connected to the metal electrode 2a.

Note that ultrasound joining is used to join the electrode and connection portion because ultrasound joining does not use heat and does not apply heat to the button battery 2 at the time of the joining. For the reason, if exposure of a battery or the like to high temperatures does not matter, spot welding may be used. Alternatively, a method using a conductive adhesive, a conductive adhesive tape, or the like may be utilized.

As shown in Fig. 3C, after the connection body 3 electrically connected to the button battery 2 and metal electrode 2a is inverted 180° in the direction S of series connection, the first connection portion 3b of the connection body 3 is joined to the metal electrode 1b of the button battery 1 by, e.g., ultrasound joining.

Note that the joining of the first connection portion 3b to the metal electrode 1b may be performed without inverting the connection body 3 electrically connected to the button battery 2 and metal electrode 2a 180° in the direction S of series connection. A reason for the use of ultrasound joining for the joining is as described above. Thus, spot welding, a method using a conductive adhesive or a conductive adhesive tape, or the like may also be utilized to join the electrode and connection portion. As a result, the first connection portion 3b is electrically connected to the metal electrode 1b, and the button batteries 1 and 2 are integrated together by the connection body 3.

As shown in Fig. 3D, the button battery 2 is moved in the radial direction R to a position where the button battery 2 faces the button battery 1 in the direction S of series connection while the button battery 1 is fixed, and the connection body 3 is folded in a Z-shape.

When the button battery 2 is pressed against the button battery 1 after the folding, the connection body 3 is compressively deformed in the direction S of series connection while remaining Z-shaped to have a shape shown in Fig. 1. Finally, the region Q is filled with the reinforcing agent 4. The battery module 100 is manufactured in the above-described manner.

As described above, the present embodiment has described that the connection body 3 is arranged between the metal electrode 1b of the button battery 1 and the metal electrode 2a of the button battery 2 and that the connection body 3 is arranged in the region Q overlapping with the button batteries 1 and 2 when the battery module 100 is viewed in a plan view from, e.g., the metal electrode 2b of the button battery 2 in the direction S of series connection.

With the above-described configuration, a portion of the connection body is prevented from protruding outward in the radial direction R from the outer circumferences of the button batteries 1 and 2 as in a conventional battery module. An outer diameter of the battery module 100 is equal to outer diameters of the button batteries 1 and 2.

Thus, the battery module 100 including a configuration which allows achievement of reduction in diameter in the radial direction R of the battery module 100 to achieve size reduction can be provided.

### (Second Embodiment)

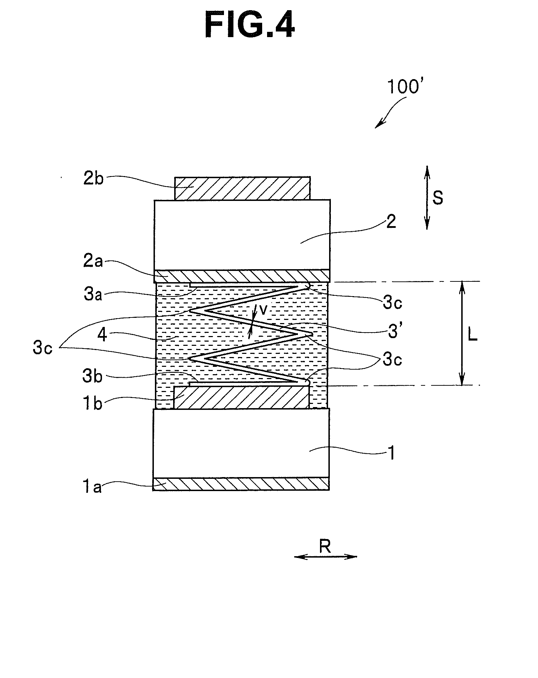

Fig. 4 is a view schematically showing a configuration of a battery module illustrating the present embodiment.

The configuration of the battery module according to the second embodiment is different from the above-described battery module 100 according to the first embodiment shown in Figs. 1 and 2 in that the number of folded portions 3c which are formed in a connection body 3 is larger than the number of folded portions 3c in the first embodiment. Accordingly, only the difference will be described, same components as in the first embodiment are denoted by same reference numerals, and a description of the components will be omitted.

As shown in Fig. 4, the connection body 3' in a battery module 100' according to the present embodiment has a plurality of (e.g., five in Fig. 4) folded portions 3c along a direction S of series connection between a first connection portion 3b and a second connection portion 3a. The connection body 3' is formed by being mountain-folded a plurality of times and being valley-folded a plurality of times between a button battery 1 and a button battery 2. Note that the number of folded portions 3c is not limited to five.

A distance between the button batteries 1 and 2 in the direction S of series connection increases each time the number of folded portions 3c increases by one and decreases each time the number of folded portions 3c decreases by one. The distance between the button batteries 1 and 2 in the direction S of series connection increases with increase in an angle constituting an acute angle of each folded portion 3c in a cross section in Fig. 4, i.e., a folded angle and decreases with decrease in the folded angle.

That is, a distance L between the button batteries 1 and 2 in the direction S of series connection can be freely set according to the number of folded portions 3c of the connection body 3' and/or folded angles.

As shown in Fig. 4, the connection body 3' is arranged between a metal electrode 1b of the button battery 1 and a metal electrode 2a of the button battery 2 and is arranged in a region which overlaps with the individual batteries 1 and 2 when the battery module 100' is viewed in a plan view from, e.g., a metal electrode 2b of the button battery 2 in the direction S of series connection, like the above-described first embodiment.

Note that remaining components of the battery module 100' are same as the corresponding components of the battery module 100 according to the above-described first embodiment and that a description of the components will be omitted.

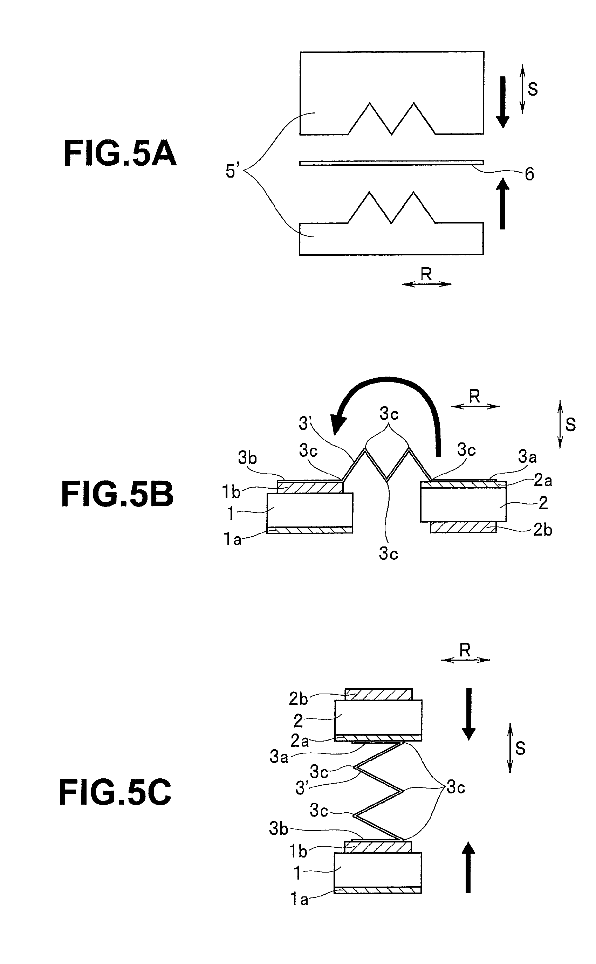

A method for manufacturing the above-described battery module 100' will be described with reference to Figs. 5A to 5C. Fig. 5A is a view schematically showing a state in which a plate-like member is set in a die in a process showing the method for manufacturing the battery module in Fig. 4. Fig. 5B is a view schematically showing a state in which a first connection portion of the plate-like member in Fig. 5A deformed by the die is electrically connected to a metal electrode of one button battery and a second connection portion is electrically connected to a metal electrode of the other button battery in the process showing the method for manufacturing the battery module in Fig. 4. Fig. 5C is a view schematically showing a state in which the other button battery in Fig. 5B is moved until the other button battery faces the one button battery in the process showing the method for manufacturing the battery module in Fig. 4.

To manufacture the battery module 100', a plate-like member 6 made of, e.g., aluminum is first set in a die 5', as shown in Fig. 5A. After the setting, the plate-like member 6 is mountain-folded and valley-folded using the die 5' to have a plurality of (e.g., five) folded portions 3c. As a result, the plate-like member 6 forms the connection body 3'.

As shown in Fig. 5B, in a state in which the button battery 1 and button battery 2 are placed so as to be coplanar with each other, the second connection portion 3 a of the connection body 3' is joined to the metal electrode 2a of the button battery 2 by, e.g., ultrasound joining, and the first connection portion 3b of the connection body 3' is joined to the metal electrode 1b of the button battery 1 by, e.g., ultrasound joining.

Note that the button batteries 1 and 2 are placed so as to be coplanar with each other because the joining of the second connection portion 3 a to the metal electrode 2a and the joining of the first connection portion 3b to the metal electrode 1b that are performed together facilitate joining work to allow improvement in workability.

The joining of the electrodes and connection portions in Fig. 5B may be performed by utilizing spot welding, a method using a conductive adhesive or a conductive adhesive tape, or the like, as in the above-described first embodiment.

As a result of the joining in Fig. 5B, the second connection portion 3a is electrically connected to the metal electrode 2a, and the first connection portion 3b is electrically connected to the metal electrode 1b. That is, the button battery 1 is integrated with the button battery 2 by the connection body 3.

As shown in Fig. 5C, the button battery 2 is rotationally moved 180° to a position where the button battery 2 faces the button battery 1 in the direction S of series connection while the button battery 1 is fixed. More specifically, the button battery 2 is rotationally moved to a position where the metal electrode 2a faces the metal electrode 1b.

After the rotational movement, the button battery 2 is pressed against the button battery 1, and the connection body 3' is compressively deformed in the direction S of series connection. Finally, a region Q is filled with a reinforcing agent 4. The battery module 100' shown in Fig. 4 is manufactured in the above-described manner.

As described above, in the present embodiment, the number of folded portions 3c of the connection body 3' is larger than the number of folded portions 3c of the connection body 3 according to the first embodiment.

With the above-described configuration, the distance L in the direction S of series connection of the button battery 2 from the button battery 1 can be freely set according to the number of folded portions 3c of the connection body 3' and/or folded angles.

Thus, size of the battery module 100' can be adjusted to a length suited to a storage space for a power supply portion of electronic equipment in which the battery module 100' is to be installed. Note that other effects are same as in the above-described first embodiment.

Note that the battery module 100 in the first embodiment and the battery module 100' in the second embodiment described above are each provided in electronic equipment such as a capsule endoscope.

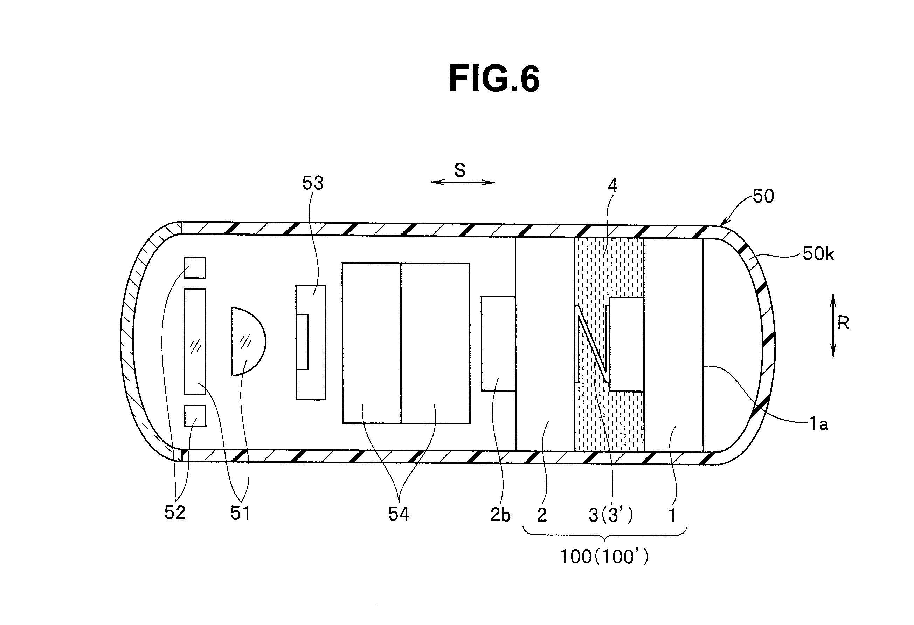

A configuration of a capsule endoscope in which the battery module in Fig. 1 or 4 is provided will be briefly described below with reference to Fig. 6. Fig. 6 is a cross-sectional view showing an outline of the configuration of the capsule endoscope in which the battery module in Fig. 1 or 4 is provided.

As shown in Fig. 6, a capsule endoscope 50 is in the form of a capsule type tablet in appearance. The capsule endoscope 50 has a housing 50k which is in the form of a capsule substantially elliptical in vertical cross section and is made of, e.g., resin. A front portion of the housing 50k is formed of a transparent member.

Inside the housing 50k, lenses 51 for observing an interior of a body cavity, a light source 52 which illuminates the interior of the body cavity and is made up of, e.g., an LED, a solid image pickup device 53 which subjects light incidence to a light-receiving surface via the lenses 51 to predetermined photoelectric conversion processing and outputs an image signal and is made up of, e.g., a CCD or CMOS sensor, a peripheral circuit board 54 which has peripheral circuits such as a driver circuit which controls driving of the image pickup device 53, a communication circuit which transfers and outputs a picked-up image signal to outside the body cavity, and a control circuit which controls driving of the entire capsule endoscope 50 and is made up of, e.g., a rigid board, and the above-described battery module 100 (100') are provided.

Note that the capsule endoscope 50 is configured such that electrical power is supplied from the battery module 100 (100') to the light source 52, solid image pickup device 53, and peripheral circuit board 54 via a flexible board (not shown) which is provided in the capsule endoscope 50 when the flexible board or the like is electrically connected to the metal electrode 1a of the button battery 1 and the metal electrode 2b of the button battery 2.

The battery module 100 (100') is provided in the capsule endoscope 50 such that a radial direction R coincides with a short axis direction of the capsule endoscope 50.

As described above, since the connection body 3 (3') is arranged between the button batteries 1 and 2 so as not to protrude from the outer circumferences of the individual button batteries 1 and 2, the battery module 100 (100') has a diameter reduced in the radial direction R.

An inner diameter in the short axis direction of the capsule endoscope 50 can thus be made to coincide with the diameters in the radial direction R of the individual button batteries 1 and 2, which can achieve reduction in size of the capsule endoscope 50.

Since the connection body 3 (3') does not protrude from the outer circumferences of the individual button batteries 1 and 2, the battery module 100 (100') can be easily incorporated into the capsule endoscope 50.

A reason for the easy incorporation is that a portion of the connection body 3 (3') does not protrude to come into contact with a different part in the capsule endoscope 50. The configuration not only improves workability but also eliminates a need to take into account insulation of the protruding connection body 3.

A configuration in which a plurality of batteries are electrically connected to one another by pressing the batteries against one another using a strong spring or the like is generally used to incorporate a battery module into electronic equipment such as a capsule endoscope. According to the configurations of the battery modules 100 and 100' of the first and second embodiments described above, electrical connection between batteries can be ensured even with a small force against the batteries. In addition, a plurality of batteries need not be incorporated one by one when the batteries are incorporated into electronic equipment such as a capsule endoscope, and the batteries can be easily incorporated, which improves workability.

Thus, the battery module 100 (100') including a configuration which allows achievement of reduction in diameter to achieve size reduction and the capsule endoscope 50 including the battery module 100 (100') can be provided.

Note that modifications will be described below. The above-described first and second embodiments have illustrated cases where the connection body 3 is composed of a plate-like member. The connection body 3 may, of course, be composed of a linear member.

Although use of aluminum (Al) as a material for the plate-like member has been illustrated, it will be appreciated that the material for the plate-like member may be a metal having high electric conductivity, such as copper (Cu) or gold (Au), or a clad material using a metal having high electric conductivity.

Use of button batteries as the one button battery 1 and the other button battery 2 has been illustrated. The batteries are not limited to primary batteries such as an alkaline dry battery and a manganese dry battery and may, of course, be secondary batteries such as a lithium ion battery, a nickel-cadmium battery, and a nickel hydrogen battery.

It will be appreciated that the number of batteries is not limited to two. The number may, of course, be three or more.

Although cases where the battery module 100 or 100' is provided in the capsule endoscope 50 have been illustrated, the present invention is not limited thereto. The present invention may, of course, be applied to a case where the battery module 100 or 100' is provided in different electronic equipment.

The above-described embodiments include inventions in various stages, and various inventions can be extracted by appropriately combining the plurality of constituent features disclosed. For example, even in a case where some constituent features are removed from all the constituent features described in the above-described one embodiment, if the problems described in Problems to be Solved by the Invention can be solved, and effects described as effects of the invention can be obtained, a configuration from which the constituent features have been removed can be extracted as an invention.

The present application claims priority from Japanese Patent Application No. 2010-253957 filed in Japan on November 12, 2010, the disclosure of which is incorporated herein by reference in the specification, the claims, and the drawings of the present application.

## Claims

1. A battery module comprising a plurality of batteries formed to have a same diameter respectively and a connection body between the plurality of batteries which electrically connects in series a metal electrode of one polarity of one of the batteries with a metal electrode of the other polarity of another battery,
wherein the connection body
includes a first connection portion which is electrically connected to the metal electrode of the one battery and a second connection portion which is electrically connected to the metal electrode of the other battery and is arranged in a region which overlaps with the individual batteries when the individual batteries connected by the connection body are viewed in a plan view from one of two sides in a direction of series connection of the individual batteries.

2. The battery module according to claim 1, wherein the connection body is composed of a plate-like member.

3. The battery module according to claim 2, wherein the connection body has a folded portion folded between the first connection portion and the second connection portion.

4. The battery module according to claim 3, wherein a distance between the one battery and the other battery in the direction of series connection is freely set according to the number of folded portions and/or a folded angle.

5. The battery module according to any one of claims 1 to 4, wherein the one battery and the other battery are integrally formed with each other by the connection body.

6. The battery module according to claim 5, wherein the region between the one battery and the other battery is filled with a reinforcing agent which reinforces connection of the first connection portion of the connection body to the metal electrode of the one battery and connection of the second connection portion of the connection body to the metal electrode of the other battery.

7. A capsule endoscope comprising a battery module, wherein the capsule endoscope comprises the battery module according to any one of claims 1 to 6.
